# FASCICULE DE BREVET EUROPEEN

(11) **EP 1 319 668 B1**
(45) Date de publication et mention de la délivrance du brevet: **27.10.2004**
(21) Numéro de dépôt: 03290606.7
(22) Date de dépôt: 12.03.2003
(51) Int. Cl.: C07K 5/06, C07K 5/02, C07D 209/42

(54) **Nouveau procédé de synthèse de dérivés de l'acide (2S, 3aS, 7aS)-1-[(S)]-alanyl]-octahydro-1H-indole-2-carboxylique et application à la synthèse du perindopril**
Verfahren zur Synthese von (2S,3aS,7aS)-1-((S)-Alanyl)-octahydro-1H-indol-2-carbonsäurederivaten und Verwendung in der Synthese von Perindopril
Method for synthesis of (2S,3aS,7aS)-1-((S)-alanyl)-octahydro-1H-indole-2-carboxylic acid derivatives and use in the synthesis of perindopril

(43) Date de publication de la demande: 18.06.2003
(73) Titulaire: Les Laboratoires Servier, 92415 Courbevoie Cedex (FR)
(72) Inventeur: Dubuffet, Thierry, 76210 Bolbec (FR); Lecouve, Jean-Pierre, 76600 Le Havre (FR)

(56) Documents cités:
- WO-A-01/58868
- WO-A-03/016336
- VINCENT M ET AL: "Stereoselective Synthesis of a New Perhydroindole Derivative of Chiral Iminodiacid, a Potent Inhibitor of Agiotensin Converting Enzyme" TETRAHEDRON, ELSEVIER SCIENCE PUBLISHERS, AMSTERDAM, NL, vol. 23, no. 16, 1982, pages 1677-1680, XP002155080 ISSN: 0040-4020

## Description

La présente invention concerne un procédé de synthèse industrielle des composés de formule (I) : 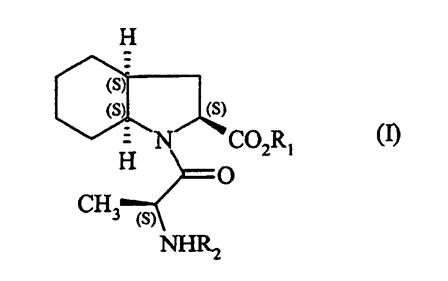 dans laquelle R₁ représente un atome d'hydrogène ou un groupement alkyle (C₁-C₆) linéaire ou ramifié ou benzyle, et R₂ représente un groupement protecteur de la fonction amino,
et leur application à la synthèse industrielle du perindopril de formule (II) : 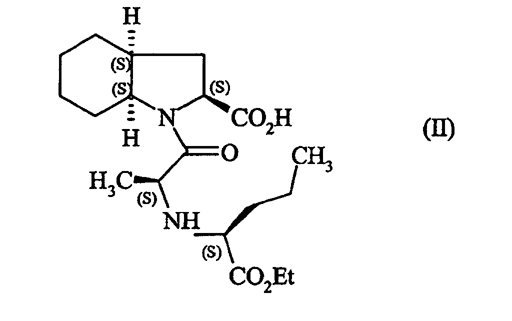 et de ses sels pharmaceutiquement acceptables

Le perindopril, ainsi que ses sels pharmaceutiquement acceptables, et plus particulièrement son sel de tert-butylamine, possèdent des propriétés pharmacologiques intéressantes.
Leur principale propriété est d'inhiber l'enzyme de conversion de l'angiotensine I (ou kininase II), ce qui permet d'une part d'empêcher la transformation du décapeptide angiotensine I en octapeptide angiotensine Il (vasoconstricteur), et d'autre part de prévenir la dégradation de la bradykinine (vasodilatateur) en peptide inactif.

Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte-tenu de l'intérêt pharmaceutique de ce composé, il était important de pouvoir y accéder avec un procédé de synthèse industrielle performant, conduisant au perindopril avec un bon rendement et avec une excellente pureté, à partir de matières premières bon marché et aisément accessibles.

La demande de brevet EP 1 256 590 décrit déjà un procédé de préparation des composés de formule (I).
Toutefois, celui-ci présente l'inconvénient d'utiliser comme matière première un ester de l'acide (2S)-dihydroindole-2-carboxylique, qui n'est pas commercial et dont la préparation nécessite plusieurs étapes de synthèse (dont une étape de résolution) à partir de l'acide indole-2-carboxylique.

La demanderesse a présentement mis au point un nouveau procédé de synthèse industrielle des dérivés de formule (I) qui présente l'avantage d'utiliser comme seules sources de chiralité l'alanine, matière première naturelle et donc peu coûteuse, et un dérivé aisément accessible de la sérine.

Plus spécifiquement, la présente invention concerne un procédé de synthèse industrielle du composé de formule (I): 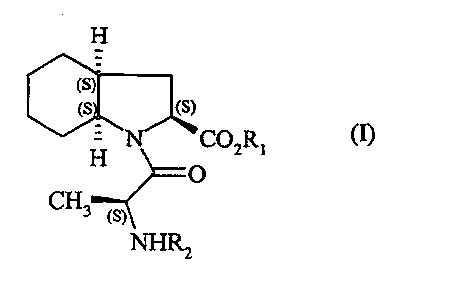 dans laquelle R₁ représente un atome d'hydrogène ou un groupement alkyle (C₁-C₆) linéaire ou ramifié ou benzyle, et R₂ représente un groupement protecteur de la fonction aminé,
caractérisé en ce que l'on fait réagir la 2,7-oxepanedione de formule (III) : 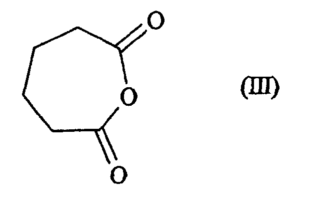 avec le composé de formule (IV) : 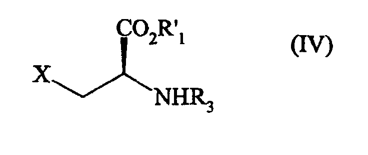 dans laquelle R'₁ représente un groupement benzyle ou alkyle (C₁-C₆) linéaire ou ramifié, R₃ représente un groupement protecteur de la fonction amine qui est différent de R'₁, et X représente un atome de brome ou d'iode,
en présence de zinc ou d'amalgame zinc/cuivre,
pour conduire après déprotection de la fonction aminé au composé de formule (V): 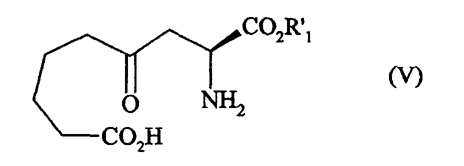 dans laquelle R'₁ est tel que défini précédemment,
que l'on cyclise par réaction avec un agent de chloration tel que le chlorure de thionyle ou le chlorure d'oxalyle, ou avec un agent de couplage peptidique, pour conduire au composé de formule (VI) : 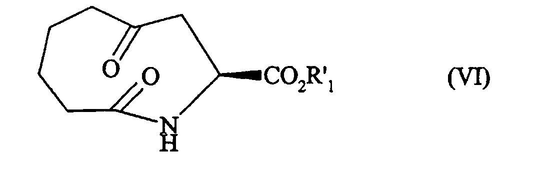 dans laquelle R'₁ est tel que défini précédemment,
que l'on soumet à une réaction de couplage en présence de titane,
pour conduire au composé de formule (VII) : 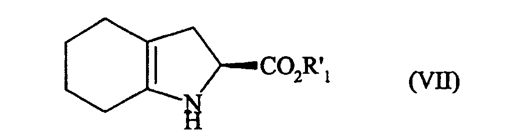 dans laquelle R'₁ est tel que défini précédemment,
que l'on met en réaction, soit avec le dérivé de l'alanine de formule (VIIIa) 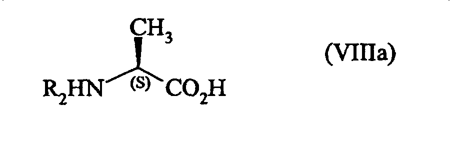 dans laquelle R₂ est tel que défini dans la formule (I),
dans un solvant organique tel que, par exemple, le tétrahydrofurane ou l'acétate d'éthyle, en présence d'une quantité de dicyclohexylcarbodiimide comprise entre 1 et 1,2 mole par mole de composé de formule (VII) utilisé, d'une quantité de triéthylamine comprise entre 1 et 1,2 mole par mole de composé de formule (VII) utilisé, et en présence éventuelle de 1-hydroxybenzotriazole,
à une température comprise entre 20 et 50°C,
soit avec le chlorure d'acide de formule (VIIIb) : 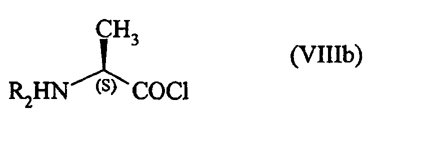 dans laquelle R₂ est tel que défini dans la formule (I),
pour conduire après isolement puis recristallisation au composé de formule (IX) : 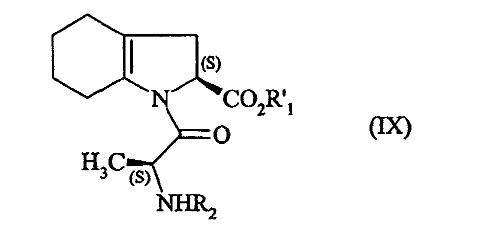 dans laquelle R'₁ et R₂ sont tels que définis précédemment,
que l'on hydrogène en présence d'un catalyseur tel que, par exemple, le palladium, le platine, le rhodium ou le nickel,
sous une pression d'hydrogène comprise entre 1 et 30 bars, de préférence entre 1 et 10 bars , pour conduire, après éventuelle déprotection ou reprotection de la fonction acide, au composé de formule (I).

Le composé de formule (I) ainsi obtenu est ensuite soumis, le cas échéant, à une réaction de déprotection des fonctions acide et amine, suivie d'une réaction de couplage, soit avec le 2-oxo-pentanoate d'éthyle dans des conditions d'amination réductrice,
soit avec un composé de formule (X) : 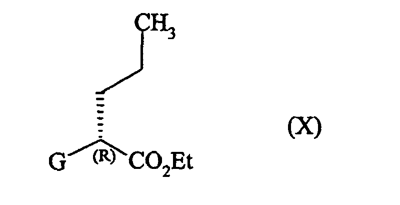 dans laquelle G représente un groupement partant choisi parmi atome d'halogène,

―O―SO₂CH₃

et 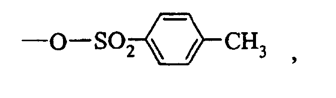 pour conduire au perindopril optiquement pur, que l'on transforme, si on le souhaite, en un sel pharmaceutiquement acceptable tel que le sel de tert-butylamine.

Parmi les groupements protecteurs de la fonction amine utilisables dans le procédé de la présente invention, on peut citer à titre non limitatif les groupements tert-butyloxycarbonyle et benzyle.

L'exemple ci-dessous illustre l'invention, mais ne la limite en aucune façon.

### EXEMPLE : Acide (2S, 3aS, 7aS)-1{(2S)2[(tert-butyloxycarbonyl)-amino]-propionyl}-octahydro-1H-indole-2-carboxylique

### Stade A : Acide (8S)-9-benzyloxy-8-[(tert-butyloxycarbonyl)-amino]-6,9-dioxo-nonanoïque

Dans un réacteur, placer 200 g de 2,7-oxepanedione, 1 l de diméthylformamide, 204 g de poudre de zinc puis 758 g de (2S)-2-[(tert-butyloxycarbonyl)-amino]-3-iodopropanoate de benzyle. Laisser la température du mélange réactionnel monter sous agitation à 60°C, puis maintenir cette température pendant 1 h. Ramener ensuite le mélange réactionnel à température ambiante, le filtrer puis le verser sur une solution glacée d'acide chlorhydrique à 5 %, puis extraire par l'acétate d'éthyle et évaporer à sec.
L'acide (8S)-9-benzyloxy-8-[(tert-butyloxycarbonyl)-amino]-6,9-dioxo-nonanoïque est ainsi obtenu avec un rendement de 80 %.

### Stade B : Acide (8S)-8-amino-9-benzyloxy-6, 9-dioxo-nonanoïque

Dans un réacteur, placer 200 g du composé obtenu dans le stade précédent, 1,5 l de dichlorométhane et 56 g d'acide trifluoroacétique. Après 1h30 d'agitation à température ambiante, ajouter 2 l d'une solution saturée d'hydrogénocarbonate de sodium. Extraire par le dichlorométhane et évaporer à sec.

L'acide (8S)-8-amino-9-benzyloxy-6,9-dioxo-nonanoïque est ainsi obtenu avec un rendement de 90 %.

### Stade C : (2S)-4,9-Dioxo-2-azonanecarboxylate de benzyle

Dans un réacteur, placer 200 g du composé obtenu dans le stade précédent, 2 l d'acétate d'éthyle puis 44 g de 1-hydroxybenzotriazole et 140 g de dicyclohexylcarbodiimide. Le mélange est ensuite porté à 30°C pendant 3 heures, puis il est refroidi et filtré. Le filtrat est ensuite lavé, puis évaporé à sec.
Le (2S)-4,9-dioxo-2-azonanecarboxylate de benzyle est ainsi obtenu avec un rendement de 95 %.

### Stade D: (2S)-2,3,4,5,6,7-Hexahydro-1H-indole-2-carboxylate de benzyle, paratoluènesulfonate

Dans un réacteur, placer 2 l d'une suspension de titane sur graphite 0,7 M dans le tétrahydrofurane, puis amener au reflux et ajouter lentement une solution de 200 g du composé obtenu dans le stade précédent dans 2 l de tétrahydrofurane. Amener ensuite le mélange réactionnel à température ambiante, le filtrer sur un lit de silice puis laver et évaporer le filtrat à sec. Reprendre le produit brut dans le toluène, ajouter 131 g d'acide paratoluènesulfonique monohydrate, porter la suspension au reflux et éliminer l'eau par distillation azéotropique. Refroidir le milieu à température ambiante, filtrer, laver le sel par du toluène puis le sécher.
Le paratoluènesulfonate du (2S)-2,3 ,4,5 ,6,7-hexahydro- 1*H*-indole-2-carboxylate de benzyle est ainsi obtenu avec un rendement de 77 %.

### Stade E : (2S)-1-{(2S)-2-[(Tert-butyloxycarbonyl)-amino]-propionyl}-2,3-dihydro-1H-indole-2-carboxylate de benzyle

Dans un réacteur sous agitation sont introduits 200 g du composé obtenu dans le stade précédent, 46 g de triéthylamine, 1 l de tétrahydrofurane puis, après 10 mn d'agitation à température ambiante, 88 g de N-[tert-butyloxycarbonyl]-(S)-alanine, et 100 g de dicylohexylcarbodiimide. Le mélange hétérogène est ensuite agité à température ambiante pendant 6 heures, puis il est refroidi à 0°C et filtré.
Le filtrat est ensuite lavé, puis recristallisé dans un mélange hexane/acétate d'éthyle 10/1 pour conduire au produit attendu avec un rendement de 81% et une pureté chimique de 98%.

### Stade F : Acide (2S, 3aS, 7aS)-1-{(2S)-2[(tert-butyloxycarbonyl)-amino]-propionyl} -octahydro-1H-indole-2-carboxylique

Dans un hydrogénateur, placer 200 g du composé obtenu dans le stade précédent, en solution dans l'acide acétique, puis 5 g de Pt/C à 10 %. Hydrogéner sous pression de 5 bars à température ambiante, jusqu'à absorption de la quantité théorique d'hydrogène.
Eliminer le catalyseur par filtration, puis refroidir entre 0 et 5°C et récolter le solide obtenu par filtration. Laver le gâteau et le sécher jusqu'à poids constant.
L'acide (2S, 3aS, 7aS)-1-{(2S)-2[(tert-butyloxycarbonyl)-amino]-propionyl}-octahydro-1*H*-indole-2-carboxylique est ainsi obtenu avec un rendement de 87 % et une pureté énantiomérique de 99 %.

## Revendications

1. Procédé de synthèse industrielle des composés de formule (I): 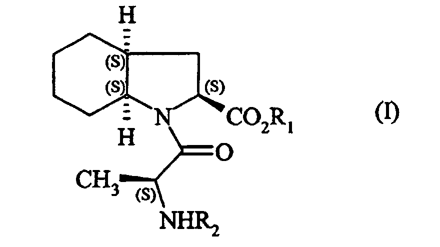 dans laquelle R₁ représente un atome d'hydrogène ou un groupement alkyle (C₁-C₆) linéaire ou ramifié ou benzyle, et R₂ représente un groupement protecteur de la fonction amine,
**caractérisé en ce que** l'on fait réagir la 2,7-oxepanedione de formule (III) : 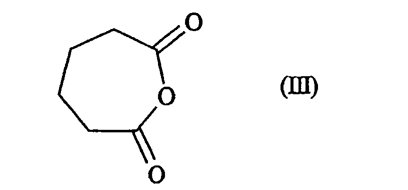 avec le composé de formule (IV) : 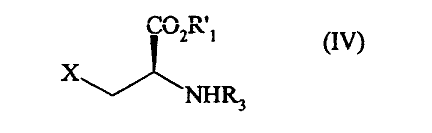 dans laquelle R'₁ représente un groupement benzyle ou alkyle (C₁-C₆) linéaire ou ramifié, R₃ représente un groupement protecteur de la fonction aminé qui est différent de R'₁, et X représente un atome de brome ou d'iode,
en présence de zinc ou d'amalgame zinc/cuivre,
pour conduire après déprotection de la fonction aminé au composé de formule (V) : 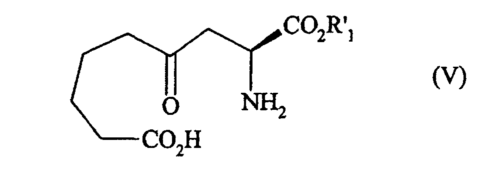 dans laquelle R'₁ est tel que défini précédemment,
que l'on cyclise par réaction avec un agent de chloration tel que le chlorure de thionyle ou le chlorure d'oxalyle, ou avec un agent de couplage peptidique, pour conduire au composé de formule (VI) : 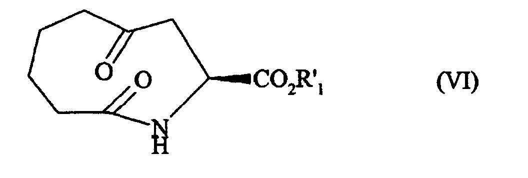 dans laquelle R'₁ est tel que défini précédemment,
que l'on soumet à une réaction de couplage en présence de titane,
pour conduire au composé de formule (VII) : 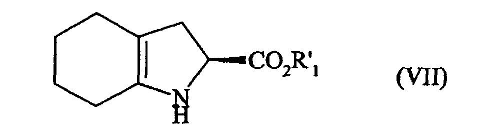 dans laquelle R'₁ est tel que défini précédemment,
que l'on met en réaction, soit avec le dérivé de l'alanine de formule (VIII) : 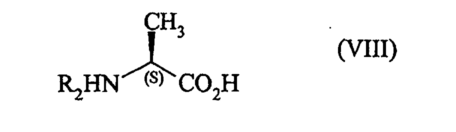 dans laquelle R₂ est tel que défini dans la formule (I),
dans un solvant organique tel que, par exemple, le tétrahydrofurane ou l'acétate d'éthyle, en présence d'une quantité de dicyclohexylcarbodiimide comprise entre 1 et 1,2 mole par mole de composé de formule (VII) utilisé, d'une quantité de triéthylamine comprise entre 1 et 1,2 mole par mole de composé de formule (VII) utilisé, et en présence éventuelle de 1-hydroxybenzotriazole,
à une température comprise entre 20 et 50°C,
soit avec le chlorure d'acide de formule (VIIIb) : 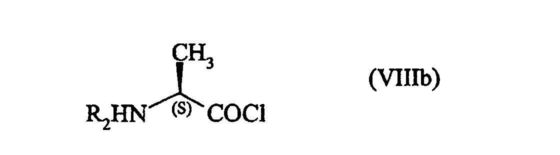 dans laquelle R₂ est tel que défini dans la formule (I),
pour conduire après isolement puis recristallisation au composé de formule (IX) : 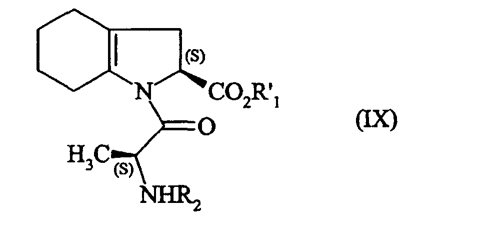 dans laquelle R'₁ et R₂ sont tels que définis précédemment,
que l'on hydrogène en présence d'un catalyseur tel que, par exemple, le palladium, le platine, le rhodium ou le nickel,
sous une pression d'hydrogène comprise entre 1 et 30 bars, pour conduire, après éventuelle déprotection ou reprotection de la fonction acide, au composé de formule (I).

2. Procédé de synthèse selon la revendication 1, **caractérisé en ce que** la pression d'hydrogène de la réaction d'hydrogénation est comprise entre 1 et 10 bars.

3. Procédé de synthèse selon la revendication 1, permettant l'obtention du dérivé de formule (I) dans laquelle R₁ représente un atome d'hydrogène et R₂ représente un groupement tert-butyloxycarbonyle.

4. Procédé de synthèse du perindopril ou de ses sels pharmaceutiquement acceptables à partir d'un composé de formule (I), **caractérisé en ce que** ledit composé de formule (I) est obtenu selon le procédé de la revendication 1.

## Patentansprüche

1. Verfahren zur technischen Synthese der Verbindungen der Formel (I): 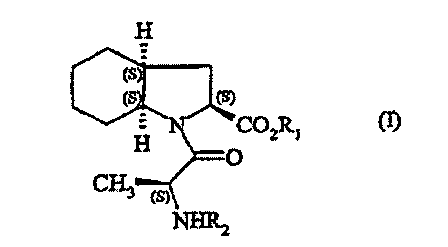 in der R₁ ein Wasserstoffatom oder eine geradkettige oder verzweigte (C₁-C₆)-Alkylgruppe oder Benzylgruppe und R₂ eine Schutzgruppe für die Aminfunktion bedeuten,
**dadurch gekennzeichnet, daß** man 2,7-Oxepandion der Formel (III): 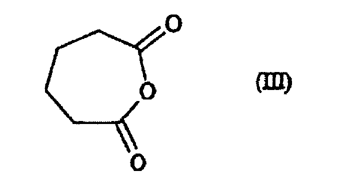 mit der Verbindung der Formel (IV): 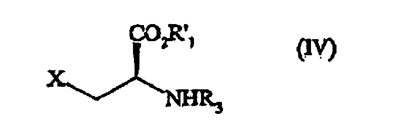 in der R'₁ eine Benzylgruppe oder eine geradkettige oder verzweigte (C₁-C₆)-Alkylgruppe, R₃ eine Schutzgruppe für die Aminfunktion, die von R'₁ verschieden ist, und X ein Brom- oder Iodatom bedeuten,
in Gegenwart von Zink oder Zink/Kupfer-Amalgam umsetzt,
so daß man nach der Abspaltung der Schutzgruppe der Aminfunktion die Verbindung der Formel (V) erhält: 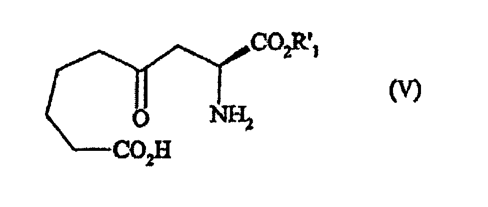 in der R'₁ die oben angegebenen Bedeutungen besitzt,
welche man durch Umsetzung mit einem Chlorierungsmittel, wie Thionylchlorid oder Oxalylchlorid, oder einem Peptid-Kupplungsmittel cyclisiert zur Bildung der Verbindung der Formel (VI): 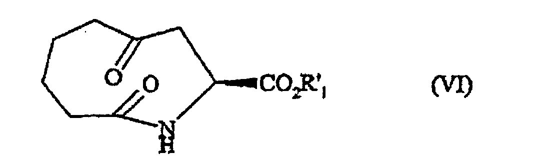 in der R'₁ die oben angegebenen Bedeutungen besitzt,
welche man einer Kupplungsreaktion in Gegenwart von Titan unterwirft,
zur Bildung der Verbindung der Formel (VII): 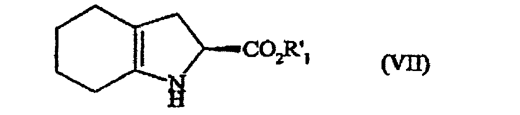 in der R'₁ die oben angegebenen Bedeutungen besitzt,
welche man entweder mit dem Anilin-Derivat der Formel (VIII): 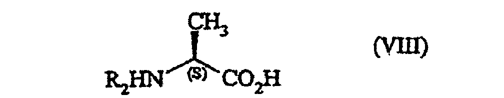 in der R₂ die bezüglich der Formel (I) angegebenen Bedeutungen besitzt,
in einem organischen Lösungsmittel, wie beispielsweise Tetrahydrofuran oder Ethylacetat, in Gegenwart einer Dicyclohexylcarbodiimid-Menge zwischen 1 und 1,2 Mol pro Mol der verwendeten Verbindung der Formel (VII), einer Triethylamin-Menge zwischen 1 und 1,2 Mol pro Mol der verwendeten Verbindung der Formel (VII) und gegebenenfalls in Gegenwart von 1-Hydroxybenzotriazol bei einer Temperatur zwischen 20 und 50°C umsetzt,
oder mit dem Säurechlorid der Formel (VIIIb): 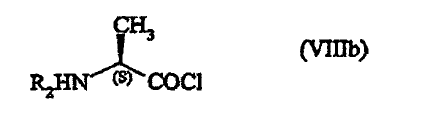 in der R₂ die bezüglich der Formel (I) angegebenen Bedeutungen besitzt, umsetzt,
so daß man nach der Isolierung und der Umkristallisation die Verbindung der Formel (IX) erhält: 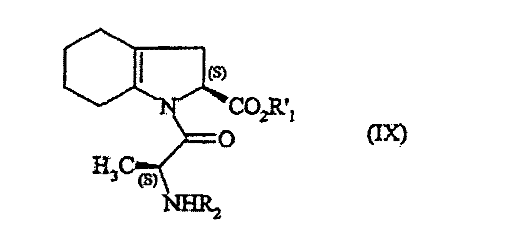 in der R'₁ und R'₂ die oben angegebenen Bedeutungen besitzen,
welche man in Gegenwart eines Katalysators, wie beispielsweise Palladium, Platin, Rhodium oder Nickel,
bei einem Wasserstoffdruck zwischen 1 und 30 bar hydriert, so daß man nach der eventuellen Abspaltung der Schutzgruppe oder den erneuten Schutz der Säurefunktion die Verbindung der Formel (I) erhält.

2. Syntheseverfahren nach Anspruch 1, **dadurch gekennzeichnet, daß** der Wasserstoffdruck der Hydrierreaktion zwischen 1 und 10 bar liegt.

3. Syntheseverfahren nach Anspruch 1 zur Herstellung des Derivats der Formel (I), in der R₁ ein Wasserstoffatom und R₂ eine tert.-Butyloxycarbonylgruppe bedeuten.

4. Verfahren zur Synthese von Perindopril oder von seinen pharmazeutisch annehmbaren Salzen, ausgehend von einer Verbindung der Formel (I), **dadurch gekennzeichnet, daß** die Verbindung der Formel (I) nach dem Verfahren nach Anspruch 1 erhalten worden ist.

## Claims

1. Process for the industrial synthesis of compounds of formula (I) : 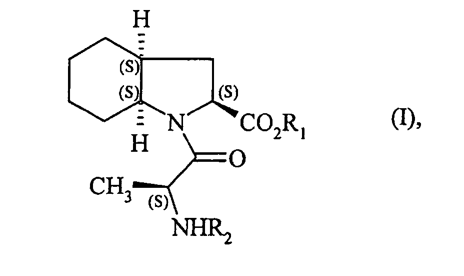 wherein R₁ represents a hydrogen atom or a linear or branched (C₁-C₆)alkyl or benzyl group and R₂ represents a protecting group for the amine function,
**characterised in that** 2,7-oxepanedione of formula (III) : 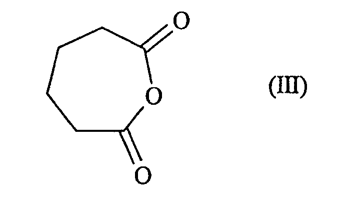 is reacted with the compound of formula (IV) : 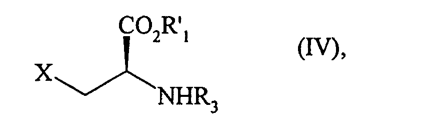 wherein R'₁ represents a benzyl or linear or branched (C₁-C₆)alkyl group, R₃ represents a protecting group for the amine function which is different from R'₁, and X represents a bromine or iodine atom,
in the presence of zinc or zinc/copper amalgam,
to yield, after deprotection of the amine function, the compound of formula (V) : 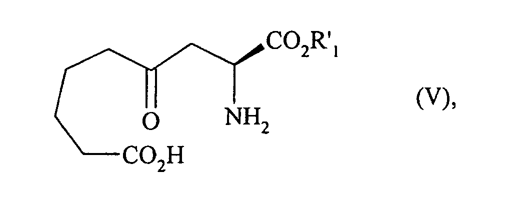 wherein R'₁ is as defined hereinbefore,
which is cyclised by reaction with a chlorination reagent such as thionyl chloride or oxalyl chloride or with a peptide coupling agent to yield the compound of formula (VI) : 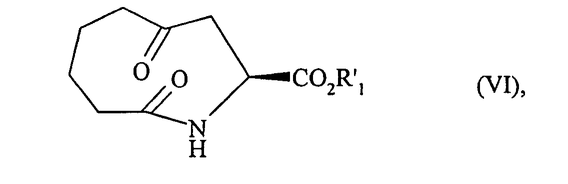 wherein R'₁ is as defined hereinbefore,
which is subjected to a coupling reaction in the presence of titanium
to yield the compound of formula (VII) : 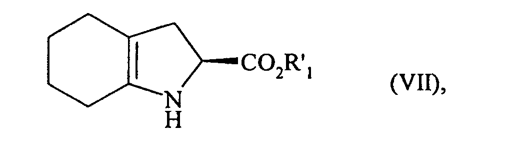 wherein R'₁ is as defined hereinbefore,
which is reacted either with the alanine compound of formula (VIII) : 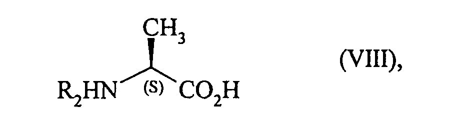 wherein R₂ is as defined for formula (I),
in an organic solvent such as, for example, tetrahydrofuran or ethyl acetate,
in the presence of an amount of dicyclohexylcarbodiimide of from 1 to 1.2 mol per mol of compound of formula (VII) used and an amount of triethylamine of from 1 to 1.2 mol per mol of compound of formula (VII) used and optionally in the presence of 1-hydroxybenzotriazole,
at a temperature of from 20 to 50°C,
or with the acid chloride of formula (VIIIb) : 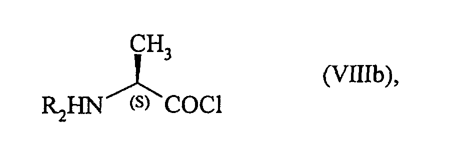 wherein R₂ is as defined for formula (I),
to yield, after isolation and then recrystallisation, the compound of formula (IX) : 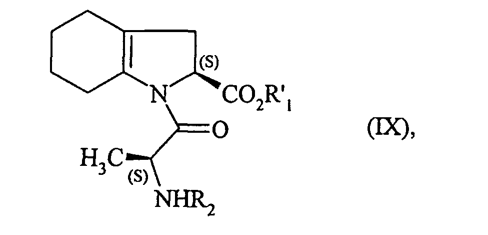 wherein R'₁ and R₂ are as defined hereinbefore,
which is hydrogenated in the presence of a catalyst such as, for example, palladium, platinum, rhodium or nickel,
under a hydrogen pressure of from 1 to 30 bars, to yield, after optional deprotection or reprotection of the acid function, the compound of formula (I).

2. Synthesis process according to claim 1, **characterised in that** the hydrogen pressure in the hydrogenation reaction is from 1 to 10 bars.

3. Synthesis process according to claim 1, allowing the compound of formula (I), wherein R₁ represents a hydrogen atom and R₂ represents a tert-butoxycarbonyl group, to be obtained.

4. Process for the synthesis of perindopril or pharmaceutically acceptable salts thereof starting from a compound of formula (I), **characterised in that** the said compound of formula (I) is obtained according to the process of claim 1.
